Europäisches Patentamt

European Patent Office

Office européen des brevets

(11) Veröffentlichungsnummer: **0 081 639**
**B1**

(12) **EUROPÄISCHE PATENTSCHRIFT**

(45) Veröffentlichungstag der Patentschrift:
26.06.85

(51) Int. Cl.⁴: **A 61 B 17/22**

(21) Anmeldenummer: 82108966.1

(22) Anmeldetag: 28.09.82

(54) Verletzungsfreie Einkoppelung und Auskoppelung von Stosswellen zu therapeutischen Zwecken.

(30) Priorität: 25.11.81 DE 3146626

(43) Veröffentlichungstag der Anmeldung:
22.06.83 Patentblatt 83/25

(45) Bekanntmachung des Hinweises auf die Patenter-teilung: 26.06.85 Patentblatt 85/26

(84) Benannte Vertragsstaaten:
CH FR GB IT LI NL

(56) Entgegenhaltungen:
DE-A- 2 913 251
DE-B- 2 722 252

(73) Patentinhaber: DORNIER SYSTEM GmbH
Postfach 1360
D-7990 Friedrichshafen (DE)

(72) Erfinder: Forssmann, Bernd, Dr. rer. nat.
Salemweg 6
D-7990 Friedrichshafen 1 (DE)
Erfinder: Hepp, Wolfgang, Dr.-Ing.
Hardtstrasse 6
D-7997 Immenstaad (DE)
Erfinder: Hoff, Günter, Dr. rer. nat.
Alpenblick 42
D-7758 Daisendorf (DE)
Erfinder: Chaussy, Christian, Prof., Dr. med.
Friedenstrasse 13a
D-8034 Germering (DE)

(74) Vertreter: Landsmann, Ralf, Dipl.-Ing.
Kleeweg 3
D-7990 Friedrichshafen 1 (DE)

## Beschreibung

Die Erfinfung betrifft eine Vorrichtung zum Zerstören von in Körpern von Lebewesen befindlichen Konkrementen mit einer an der Aussenseite des Körpers angeordneten, flüssigkeitsgefüllten Fokussierungskammer, die Teil eines Rotationsellipsoids ist und in derem einen Brennpunkt Stosswellen durch Funkenentladung erzeugt werden. Eine Anwendung ist die berührungsfreie Zerkleinerung von Nierensteinen.

Bekannt ist ein Gerät zur berührungslosen Zerkleinerung von im Körper eines Lebewesens befindlichen Konkrementen mittels Stosswellen (DE-PS 23 51 247). Stosswellen haben die Eigenschaft, an Grenzflächen zwischen Medien verschiedenen Schallwiderstandes Druck und Zug auszuüben und zum Teil reflektiert zu werden. Beim Übergang von biologischem Gewebe zu einem Nierenstein entstehen damit an der Vorder- und Rückseite des Steines Druck- und Zugbelastungen, die den Stein zerkleinern. Werden Stosswellen von aussen in den Körper eingeleitet, so bildet die Haut des Patienten gegenüber der umgebenden Luft ebenfalls eine solche Grenzfläche, an der hohe Belastungen und Reflexionen auftreten. Um Verletzungen der Haut zu vermeiden, müssen Ein- und Auskoppelstelle der Stosswellen an ein Medium grenzen, das einen ähnlichen Schallwellenwiderstand wie der Körper hat.

Bekannt ist die Lösung, den Körper in einer Wanne mit entgastem Wasser zu positionieren (Beiträge zur Urologie Band 2 Seite 64). Dadurch wird das Gerät sehr gross, die Positionierung des Patienten umständlich und die Ortung des Konkrements z. B. mittels Röntgenstrahlen erschwert.

In der DE-OS 29 13 251 ist ein Nierensteinzertrümmerer vorgeschlagen, der nach einer operativen Öffnung des Patienten direkt auf die Niere gesetzt wird. Dadurch wird der Abstand vom Gerät zum Nierenstein sehr klein, die problematische Einkoppelstelle beträgt nur wenige Quadratzentimeter. Die Einkoppelung von Stosswellen direkt in das Organ erfolgt über einen kleinen Beutel mit Wasser als Ankoppelstrecke von einigen Millimetern Dicke. Durch die bauliche Enge ist eine gleichzeitige Überwachung der Zentrierung, des richtigen Anliegens und des Erfolges der Zertrümmerung nicht möglich. Das Problem der verletzungsfreien Auskoppelung der Stosswellen aus dem Körper — sie geben ja nicht ihre gesamte Energie an den Stein ab — ist in dieser DE-OS nicht behandelt.

Aufgabe der Erfindung ist es, ein Gerät zum berührungsfreien und nichtinvasiven Zerkleinern von im Körper von Lebewesen befindlichen Konkrementen zu schaffen, wobei durch Funkenentladung erzeugte fokussierte Stosswellen verletzungsfrei, verlustarm und grossflächig sowohl in den Körper eingekoppelt als auch aus dem Körper ausgekoppelt werden und gleichzeitig eine Überwachung des richtigen Anliegens der Zentrierung und des Erfolgs der Zerkleinerung erlaubt ist.

Die Aufgabe wird dadurch gelöst, dass sich zwischen der Fokussierungskammer und der Einkoppelstelle in den Körper ein Kissen befindet, welches mit entgastem Wasser gefüllt ist und dessen Vorderseite und Rückseite aus einer formanpassungsfähigen, akustisch angepassten, dünnen Folie besteht, wobei das Kissen durch seine Flexibilität, seine geringe Dicke und die Verwendung durchlässiger Materialien eine gleichzeitige Einleitung von Röntgen- oder Ultraschallwellen zur Ortung der Konkremente und zur Überwachung des Erfolgs der Zerkleinerung erlaubt, und daß sich an der Auskoppelstelle aus dem Körper ein dem ersten ähnliches zweites Wasserkissen befindet, welches grossflächig den Körper umfassend ausgebildet ist und dessen Rückseite über eine dämpfende oder diffus reflektierende Schicht (z. B. Schaumgummi) an einer aus einem oder mehreren Teilen bestehenden starren Schale befestigt ist.

Die erfindungsgemässe Vorrichtung bietet folgende Vorteile :

— Verzicht auf operativen Eingriff,
— Verzicht auf eine grosse Wanne und Positionierungsvorrichtung für den Patienten, die die Zugänglichkeit zum Patienten erschweren,
— einfaches Positionieren auf das Konkrement, da das flexible Wasserkissen eine Verschiebung der Fokussierungskammer in alle Richtungen erlaubt,
— einfache Ortung des Konkrements und Erfolgskontrolle mit Röntgenstrahlen oder Ultraschall, da die Vorlaufstrecke durch Zusammenpressen des flexiblen Kissens minimiert werden kann.

Ausgestaltungen der Erfindung sind Gegenstände von Unteransprüchen.

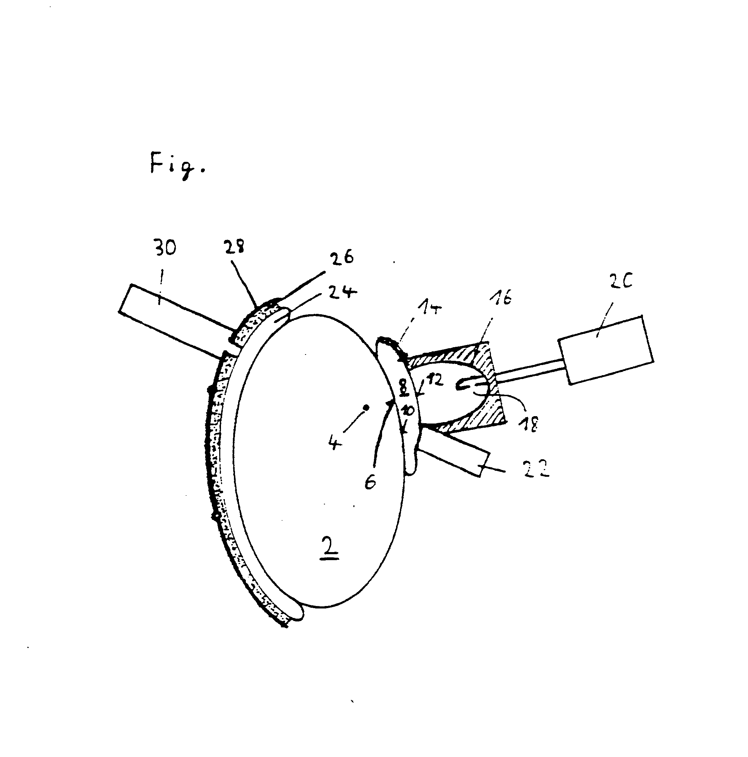

Weitere Vorteile und Anwendungsmöglichkeiten ergeben sich aus der einzigen Figur, die nachfolgend beschrieben ist.

Die Figur zeigt eine Ausbildung der erfindungsgemässen Vorrichtung.

Die Figur zeigt im Querschnitt einen menschlichen Körper 2, in dem sich ein Konkrement 4, z. B. ein Nierenstein, befindet. Auf der dem Konkrement 4 am nächsten liegenden Hautstelle 6 liegt luftspaltfrei ein flüssigkeitsgefülltes Kissen 8, dessen Vorderseite 10 und Rückseite 12 aus dünnen, flexiblen, akustisch angepassten Membranen bestehen. Seitlich besitzt das Kissen ein Sichtfenster 14 zum Beobachten des luftspaltfreien Anliegens der Membran 10 an die Körperstelle 6. An die Membran 12 schliesst sich eine flüssigkeitsgefüllte, ellipsoide Fokussierungskammer 16 mit einer Funkenstrecke 18 in einem ihrer Brennpunkte an. Die elektrische Schaltung zur Stromversorgung der Funkenstrecke ist im Gehäuse 20 zusammengefasst und nicht Gegenstand dieser Erfindung. Das flexible Kissen erlaubt eine solche Positionierung der Fokussierungskammer, dass der zweite Brennpunkt der Fokussierungskammer mit dem Konkrement 4 zusammenfällt. Zu dieser Positionierung dienen mindestens zwei Röntgenröhren, die sich am Kissen 8 befinden. In der Zeichenebene ist dies die Röhre 22. Auf der gegenüberliegenden Körperseite befindet sich ein zweites flüssigkeitsgefülltes Kissen 24, welches einen grösseren Körperbereich umfasst. Dieses Kissen ist über eine dämpfende Schicht 26, die z. B. aus Schaumgummi bestehen kann, an einer starren Schale 28 befestigt. Die Schale 28 besteht hier aus mehreren zueinander beweglichen Teilen. Jeweils koaxial zu den Röntgenröhren befinden sich am zweiten Kissen Bildverstärker, in der Zeichenebene ist dies der Bildverstärker 30.

Zur Zerstörung eines Konkrements 4 wird dieses mittels der beiden Röntgenröhren 22 und Bildverstärker 30 geortet. Die Vorrichtung wird so positioniert, dass das Konkrement im zweiten Brennpunkt der Fokussierungskammer 16 liegt. Durch Zündung der Funkenstrecke 18 werden Stosswellen erzeugt. Diese werden von der Fokussierungskammer 16 auf das Konkrement 4 geleitet. Durch das akustisch angepasste Kissen 8 gelingt eine verletzungsfreie und verlustarme Einkoppelung in den Körper 2. Die Stosswellen geben nicht ihre gesamte Energie an das Konkrement 4 ab, sondern laufen — allerdings geschwächt — durch den Körper weiter. Das dem Körper 2 akustisch angepasste Kissen 24 dient zur verletzungsfreien Auskoppelung der Stosswellen aus dem Körper. Ihre Energie wird hauptsächlich im Dämpfmaterial 26 und an dessen beiden Grenzflächen (24-26 und 26-28) vernichtet.

**Patentansprüche**

1. Vorrichtung zum Zerstören von im Körper eines Lebewesens befindlichen Konkrementen mit einer an der Außenseite des Körpers angeordneten, flüssigkeitsgefüllten Fokussierungskammer, die Teil eines Rotationsellipsoids ist und in derem einen Brennpunkt Stosswellen durch Funkenentladung erzeugbar sind, dadurch gekennzeichnet, dass sich zwischen der Fokussierungskammer (16) und der Einkoppelstelle (6) in den Körper (2) ein Kissen (8) befindet, welches mit entgastem Wasser gefüllt ist und dessen Vorderseite (10) und Rückseite (12) aus formanpassungsfähigen, akustisch angepassten dünnen Folien bestehen, wobei das Kissen durch seine Flexibilität, seine geringe Dicke und die Verwendung durchlässiger Materialien eine gleichzeitige Einleitung von Röntgen- oder Ultraschallwellen zur Ortung der Konkremente und zur Überwachung des Erfolgs der Zerkleinerung erlaubt, und daß sich an der der Einkoppelstelle gegenüberliegenden Körperseite an der Auskoppelstelle aus dem Körper ein dem ersten ähnliches zweites Wasserkissen (24) befindet, welches grossflächig den Körper umfassend ausgebildet ist und dessen Rückseite über eine dämpfende oder diffus reflektierende Schicht (26) an einer aus einem oder mehreren Teilen bestehenden starren Schale (28) befestigt ist.

2. Vorrichtung nach Anspruch 1, dadurch gekennzeichnet, dass in die starre Schale (28) ein Eingangsschirm eines Ortungssystems (30) eingearbeitet ist.

3. Vorrichtung nach den Ansprüchen 1 und 2, dadurch gekennzeichnet, dass mindestens eines der Kissen einen oder mehrere Kontrollsensoren für das eingekoppelte Stosswellenfeld und Einrichtungen zur Erhaltung einer körpergerechten Temperatur enthält.

4. Vorrichtung nach den Ansprüchen 1 bis 3, dadurch gekennzeichnet, dass als schalleitende Substanz statt des Wassers hochviskose Flüssigkeiten oder gummiartige Substanzen (z. B. Polyurethan) verwendet werden.

5. Vorrichtung nach den Ansprüchen 1 bis 4, dadurch gekennzeichnet, dass das Kissen (8) an der Einkoppelstelle ein Fenster zum Überwachen der Zentrierung und des luftspaltfreien Anliegens seiner Vorderseite (10) an den Körper (2) besitzt.

6. Vorrichtung nach den Ansprüchen 1 bis 5, dadurch gekennzeichnet, dass die Kissen (8, 24) mittels schalleitender Paste luftspaltfrei an den Körper (2) angelegt werden.

**Claims**

1. Instrument for destroying concretions in a body of a living being, comprising a focusing chamber being liquid-filled and positioned at the outside of the body whereby the focusing chamber is a part of an ellipsoid of rotation and wherein shock waves may be generated at one focus by means of arc discharges, characterized in that a pad (8) is located between the focusing chamber (16) an the coupling site (6) of the body (2), the pad (8) is filled with degased water and the front and rear sides of the pad (10 and 12 respectively) are composed of shape-adapting acoustically matched thin foils, the pad (8), because of its flexibility, its thinness and the use of transmitting materials, permitting simultaneous introduction of X-rays or ultrasonic waves for locating the concretions and to monitor the success in comminution, and in that a second water pad (24) similar to the first one is located at the side of the body which is opposite to the coupling site, at the body decoupling site, said second pad (24) enclosing a large area of the body and having a rear side which by means of a damping or diffusely reflecting layer (26) is fastened to a rigid dish (28), consisting of one or more parts.

2. An instrument according to claim 1 characterized in that the rigid dish (28) comprises an integrated input screen of a locating system (30).

3. An instrument according to the claims 1 and 2, characterized in that at least one of the pads contains at least one monitoring sensor for the coupled field of shock waves and devices for maintaining a temperature proper for the body.

4. An instrument according to the claims 1 to 3, characterized in that highly viscous liquids or rubbery substances such as polyurethane are used instead of water as the acoustically transmitting substance.

5. An instrument according to the claims 1 to 4, characterized in that the pad (8) at the coupling site has a window for monitoring the centering and the air gap-free abutting of its front side (10) to the body (2).

6. An instrument according to the claims 1 to 5, characterized in that the pads (8, 24) are placed against the body (2) so as to exclude air gaps by means of an acoustically transmitting paste.

**Revendications**

1. Dispositif pour la destruction de concrétions se trouvant à l'intérieur du corps d'un être vivant comportant une chambre de focalisation disposée du côté extérieur du corps remplie de liquide, faisant partie d'un ellipsoïde de révolution et à l'un des foyers de laquelle peuvent être générées des ondes de choc par décharge d'étincelles, caractérisé par le fait qu'entre la chambre de focalisation (16) et le point d'application (6) sur le corps (2) se trouve un coussin (8) rempli d'eau dégazée et dont le côté avant (10) et le côté arrière (12) sont constitués de feuilles minces adaptées acoustiquement et aptes à être adaptées à différentes formes, ledit coussin permettant grâce à sa flexibilité, sa faible épaisseur et l'utilisation de matières perméables, une introduction simultanée d'ondes ultrasonores et de rayons X pour la localisation des concrétions et la surveillance du succès de leur réduction, et par le fait qu'au point de sortie situé du côté opposé du corps par rapport au point d'application, se trouve un deuxième coussin rempli d'eau (24) similaire au premier, qui entoure une grande surface du corps et dont le côté arrière est fixé, par l'intermédiaire d'une couche (26) à amortissement ou réflexion diffuse, sur une coque (28) rigide constituée en une ou plusieurs parties.

2. Dispositif selon la revendication 1, caractérisé par le fait que dans la coque rigide (28) est incorporé un écran d'entrée d'un système de localisation (30).

3. Dispositif selon les revendications 1 et 2, caractérisé par le fait que l'un au moins des coussins contient une seule ou plusieurs cellules sensibles de contrôle pour le champ d'ondes de choc appliqué ainsi que des dispositifs pour maintenir une température corporelle déterminée.

4. Dispositif selon les revendications 1 à 3, caractérisé par le fait qu'au lieu d'eau on utilise comme substance conductrice des ondes sonores des liquides à viscosité élevée ou des substances s'apparentant au caoutchouc (par exemple du polyuréthane).

5. Dispositif selon l'une des revendications 1 à 4, caractérisé par le fait que le coussin (8) au point d'application possède une fenêtre pour le contrôle du centrage et de l'application sans jeu de son côté avant (10) sur le corps (2).

6. Dispositif selon les revendications 1 à 5, caractérisé par le fait que les coussins (8, 24) s'appliquent sans jeu au corps (2) au moyen d'une pâte conductrice des ondes sonores.

Fig.

2